(19) Europäisches Patentamt
European Patent Office
Office européen des brevets

(11) Publication number: 0 475 531 B1

# (12) EUROPEAN PATENT SPECIFICATION

(45) Date of publication of patent specification: 18.01.95

(51) Int. Cl.[6]: C07C 233/55, C07C 233/63, C07C 275/42, A61K 31/245

(21) Application number: 91202310.8

(22) Date of filing: 09.09.91

## (54) Benzoic acid phenylester compounds and elastase inhibiting compositions containing the same.

(30) Priority: 10.09.90 JP 237215/90

(43) Date of publication of application: 18.03.92 Bulletin 92/12

(45) Publication of the grant of the patent: 18.01.95 Bulletin 95/03

(84) Designated Contracting States: CH DE ES FR GB IT LI

(56) References cited:
EP-A- 0 147 211
EP-A- 0 222 608
EP-A- 0 291 234

(73) Proprietor: Asahi Kasei Kogyo Kabushiki Kaisha
2-6, Dojimahama 1-chome
Kita-ku
Osaka-shi
Osaka 530 (JP)

(72) Inventor: Yamada, Hirohiko
Asahikasei-nishi-apartment No. 315, 100 Kawanarijima,
Fuji-shi,
Shizuoka (JP)
Inventor: Kouji, Hiroyuki
179-5, Nakamaru
Fuji-shi,
Shizuoka (JP)
Inventor: Hayashi, Yoshiharu
1397-307, Hirabarucho 1-chome
Nobeoka-shi,
Miyazaki (JP)

(74) Representative: Smulders, Theodorus A.H.J., Ir. et al
Vereenigde Octrooibureaux
Nieuwe Parklaan 97
NL-2587 BN 's-Gravenhage (NL)

EP 0 475 531 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel benzoic acid phenylester compounds. More particularly, it relates to benzoic acid phenylester compounds having a substituted amino group at position 4 and having inhibitory activity against human leukocyte elastase, which is a protease, and to elastase inhibiting compositions containing the same as active ingredients.

### BACKGROUND OF THE INVENTION

It is known that the activity of human leukocyte elastase, which is a protease, is associated with such diseases as pulmonary emphysema, arthritis, Crohn's disease and acute pancreatitis (see, Medicinal Research Reviews, vol 7, no. 2, pp 227-241 (1987)). Normally, leukocyte elastase in the living body functions in balance with inhibitory proteins suppressing the activity of said elastase. It is considered in the art that, in the diseases mentioned above, this balance is disturbed and the enzyme activity becomes excessively strong to cause tissue degeneration. Based on such view, investigations have recently been made extensively and intensively with a view to developing leukocyte elastase inhibitors. Thus, various leukocyte elastase inhibitors have been proposed and made subject matters of patent applications. Typical examples are those compounds that are described in JP-A-63-165357 (The term "JP-A" as used herein means an "unexamined published Japanese patent application"), JP-A-62-265221 and JP-A-1-308227. Their efficacy, however, is not satisfactory as yet.

Accordingly, it is an object of the invention to provide substances superior in leukocyte elastase inhibiting activity to the hitherto known leukocyte elastase inhibitors as well as elastase inhibiting compositions which contain said substances as active ingredients and are medically useful.

The present inventors made intensive investigations to achieve the above object and found that certain novel benzoic acid phenylester compounds have potent inhibitory activity against the human leokucyte elastase of a protease. The present invention has been completed based on this finding.

### SUMMARY OF THE INVENTION

The invention thus provides benzoic acid phenylester derivaties having a substituted amino group at position 4 and having formula [I]:

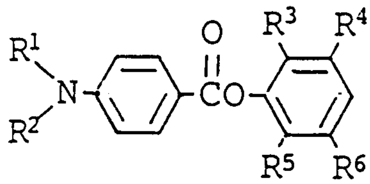

[I]

wherein $R^1$ is a $COR^7$, $CO(CR^8R^9)_nCOOH$, mesyl, benzenesulfonyl, $CONR^{10}R^{11}$, $COOCOR^{12}$ or $COOR^{13}$ group and $R^2$ is a hydrogen atom, or $R^1$ and $R^2$ combinedly represent a $-CO(CR^8R^9)_nCO-$ group for imide ring formation with the adjacent nitrogen atom; and $R^3$, $R^4$, $R^5$ and $R^6$ may be the same or different and each is a hydrogen or halogen atom or a nitrile, nitro, formyl, carboxyl, hydroxyl, $C_{1-4}$ alkoxy, benzyloxy, methyl, aminocarbonyl, trifluoromethyl, trifluoromethoxy, $C_{1-2}$ dialkylaminocarbonyloxy, $C_{1-2}$ alkoxycarbonyl, substituted or unsubstituted benzyloxycarbonyl, straight or branched $C_{1-6}$ alkylcarbonyloxy, alkyl-substituted or unsubstituted $C_{3-6}$ cycloalkylcarbonyloxy, cinnamoyloxy, straight or branched $C_{1-4}$ alkylsulfonyloxy, substituted or unsubstituted benzenesulfonyloxy, benzylsulfonyloxy, $\beta$-stylenesulfonyloxy, 2-thiophenesulfonyloxy, or straight or branched $C_{2-4}$ alkylcarbonyl group. In the above definition, $R^7$ is a straight or branched $C_{2-7}$ alkyl, straight or branched $C_{2-5}$ alkenyl, benzyl, $\alpha$-methylbenzyl, $C_{1-5}$ haloalkyl, alkyl- or carboxyl-substituted or unsubstituted $C_{3-7}$ cycloalkyl, thiol-substituted $C_{1-3}$ alkyl, methylthio-substituted $C_{1-3}$ alkyl, cyano-substituted $C_{1-5}$ alkyl, $\beta$-styryl or (2-thienyl)methyl group, $R^8$ and $R^9$ may be the same or different and each is a hydrogen atom or a straight or branched $C_{1-4}$ alkyl or phenyl group, $R^{10}$ is a straight or branched $C_{1-5}$ alkyl group, $R^{11}$ is a hydrogen atom or a straight or branched $C_{1-4}$ alkyl group, $R^{12}$ is a straight or branched $C_{1-4}$ alkyl group, $R^{13}$ is a hydroxy-substituted or unsubstituted $C_{1-4}$ alkyl, or benzyl group, and n is an integer of 1 to 3.

The invention also provides elastase inhibiting compositions containing the compounds mentioned above as active ingredients.

DETAILED DESCRIPTION OF THE INVENTION

The compounds of the invention which are represented by formula [I] can be produced, for example, by converting a 4-aminobenzoic acid phenylester compound of formula [II]:

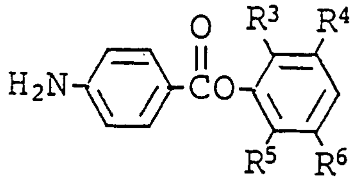

[II]

wherein $R^3$, $R^4$, $R^5$ and $R^6$ are as defined above, to a corresponding amide, imide, urea or urethane compound. The compound [II] can be readily synthesized by reacting 4-nitrobenzoyl chloride with the corresponding substituted phenol and then reducing the nitro group of the resulting ester in the manner of catalytic reduction with hydrogen in the presence of a palladium catalyst.

More specifically, the amidation and imidation can be effected by reacting the compound [II] with an acyl halide, succinic anhydride, mesyl chloride or benzenesulfonyl chloride in water or an organic solvent in the presence of an acid binding agent, such as pyridine or triethylamine, to give the corresponding compound [I] of the invention. An alternative method of amidation that can be employed comprises subjecting the compound [II] and a carboxylic acid to direct condensation in the presence of a dehydrating agent, such as DCC (1,3-dicyclohexylcarbodiimide) or EDC (1-ethyl-3-(3-dimethylaminopropyl)carbodiimide hydrochloride), without using any solvent or in an organic solvent. As regards the urea and urethane compound formation, urea compounds can be produced by reacting the compound [II] with phosgene, diphosgene, triphosgene or the like in an organic solvent in the presence of an acid binding agent, such as triethylamine, and then reacting the resulting isocyanate compound with an amine. The urethane compounds can be produced by reacting said isocyanate compound with an alcohol.

In cases where the reactive phenolic precursor contains a substituent susceptible to catalytic reduction with hydrogen, an alternative method can be used which comprises condensing a substituted aminobenzoic acid of formula [III]:

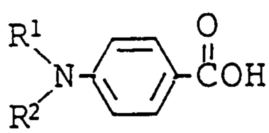

[III]

wherein $R^1$ and $R^2$ are as defined above, with a substituted phenol of formula [IV]:

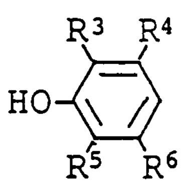

[IV]

wherein $R^3$, $R^4$, $R^5$ and $R^6$ are as defined above, in the presence of a dehydrating agent, such as DCC or EDC without using any solvent or in an organic solvent.

For a better understanding of the invention, typical examples of the compounds of formula [I] of the invention are given below. It is to be noted, however, that they are by no means limitative of the scope of the present invention. 3,5-Dichlorophenyl 4-[(1-methylcyclopropylcarbonyl)amino]benzoate, 3,5-dichlorophenyl 4-(1-methylcyclopropanecarbonylamino)benzoate, 3,5-dichlorophenyl 4-(propanoylamino)-benzoate, 3,5-dichlorophenyl 4-(butanoylamino)benzoate, 3,5-dichlorophenyl 4-(pentanoylamino)benzoate, 3,5-dichlorophenyl 4-(hexanoylamino)benzoate, 3,5-dichlorophenyl 4-(heptanoylamino)benzoate, 3,5-dichlorophenyl 4-(octanoylamino)benzoate, 3,5-dichlorophenyl 4-(isobutanoylamino)benzoate, 3,5-dichlorophenyl

4-(pivaloylamino)benzoate, 3,5-dichlorophenyl 4-(phenylacetylamino)benzoate, 3,5-dichlorophenyl 4-(trichloroacetylamino)benzoate, 3,5-dichlorophenyl 4-(trifluoroacetylamino)benzoate, 3,5-dichlorophenyl 4-(pentafluoropropanoylamino)benzoate, 3,5-dichlorophenyl 4-(propenoylamino)benzoate, 3,5-dichlorophenyl 4-(2-butenoylamino)benzoate, 3,5-dichlorophenyl 4-(2-methylpropenoylamino)benzoate, 3,5-dichlorophenyl 4-(3-methyl-2-butenonylamino)benzoate, 3,5-dichlorophenyl 4-(cyclopropanecarbonylamino)benzoate, 3,5-dichlorophenyl 4-(cyclobutanecarbonylamino)benzoate, 3,5-dichlorophenyl 4-(cyclopentanecarbonylamino)-benzoate, 3,5-dichlorophenyl 4-(cyclohexanecarbonylamino)benzoate, 3,5-dichlorophenyl 4-(3-methyl-butanoylamino)benzoate, 3,5-dichlorophenyl 4-(2-methyl-2-phenylacetylamino)benzoate, 3,5-dichlorophenyl 4-(cinnamoylamino)benzoate, 3,5-dichlorophenyl 4-(succinylamino)benzoate, 3,5-dichlorophenyl 4-(succinimidyl)benzoate, 3,5-dichlorophenyl 4-(methanesulfonylamino)benzoate, 3,5-dichlorophenyl 4-(benzenesulfonylamino)benzoate, 3,5-dichlorophenyl 4-(3-methylureido)benzoate, 3,5-dichlorophenyl 4-(3-ethylureido)benzoate, 3,5-dichlorophenyl 4-(3-propylureido)benzoate, 3,5-dichlorophenyl 4-(3-butylureido)-benzoate, 3,5-dichlorophenyl 4-(3-pentylureido)benzoate, 3,5-dichlorophenyl 4-(3-isopropylureido)benzoate, 3,5-dichlorophenyl 4-(3,3-dimethylureido)benzoate, 3,5-dichlorophenyl 4-(3-ethyl-3-methylureido)benzoate, 3,5-dichlorophenyl 4-(3-methyl-3-propylureido)benzoate, 3,5-dichlorophenyl 4-(3-butyl-3-methylureido)-benzoate, 3,5-dichlorophenyl 4-(3-isopropyl-3-methylureido)benzoate, 3,5-dichlorophenyl 4-(3-ethyl-3-butylureido)benzoate, 3,5-dichlorophenyl 4-(3,3-dibutylureido)benzoate, 3,5-dichlorophenyl 4-(methoxycarbonylamino)benzoate, 3,5-dichlorophenyl 4-(ethoxycarbonylamino)benzoate, 3,5-dichlorophenyl 4-(propoxycarbonylamino)benzoate, 3,5-dichlorophenyl 4-(isopropoxycarbonylamino)benzoate, 3,5-dichlorophenyl 4-(butoxycarbonylamino)benzoate, 3,5-dichlorophenyl 4-(benzyloxycarbonylamino)benzoate, 3,5-dichlorophenyl 4-(2-hydroxyethoxycarbonylamino)benzoate, 3,5-dichlorophenyl 4-(3-hydroxypropoxycarbonylamino)benzoate, 3,5-dichlorophenyl 4-(4-hydroxybutoxycarbonylamino)benzoate, 3,5-dichlorophenyl 4-(acetoxycarbonylamino)benzoate, 3,5-dichlorophenyl 4-(propanoyloxycarbonylamino)benzoate, 3,5-dichlorophenyl 4-(butanoyloxycarbonylamino)benzoate, 3,5-dichlorophenyl 4-(isobutanoyloxycarbonylamino)-benzoate, 3,5-dichlorophenyl 4-(2-thienylacetylamino)benzoate, 3,5-dichlorophenyl 4-(2-methyl-thiopropanoylamino)benzoate, 3,5-dichlorophenyl 4-(2-mercaptopropanoylamino)benzoate, 3,5-dichlorophenyl 4-(2,2-dimethylmalonylamino)benzoate, 3,5-dichlorophenyl 4-(2,2-dimethylsuccinylamino)-benzoate, 3,5-dichlorophenyl 4-(3,3-dimethylsuccinylamino)benzoate, 3,5-dichlorophenyl 4-(meso-2,3-dimethylsuccinylamino)benzoate, 3,5-dichlorophenyl 4-(2-phenylsuccinylamino)benzoate, 3,5-dichlorophenyl 4-(glutarylamino)benzoate, 3,5-dichlorophenyl 4-(2-hydroxycarbonylcyclobutanecarbonylamino)benzoate, 3,5-dichlorophenyl 4-(2-hydroxycarbonylcyclopentanecarbonylamino)benzoate, 3,5-dichlorophenyl 4-(2-hydroxycarbonylcyclohexanecarbonylamino)benzoate, 3,5-dichlorophenyl 4-(3-chloro-2,2-dimethyl-propanoylamino)benzoate, 3,5-dichlorophenyl 4-(2-cyanoacetylamino)benzoate, 3,5-dichlorophenyl 4-(3-cyanopropanoylamino)benzoate, 3,5-dichlorophenyl 4-(3-cyano-2,2-dimethylpropanoylamino)benzoate, 3-formyl-6-nitrophenyl 4-(tert-butoxycarbonylamino)benzoate, 6-nitrophenyl 4-(isobutanoylamino)benzoate, 5-hydroxy-6-nitrophenyl 4-(isobutanoylamino)benzoate, 3-hydroxy-6-nitrophenyl 4-(isobutanoylamino)-benzoate, 5-methoxy-6-nitrophenyl 4-(isobutanoylamino)benzoate, 2-nitro-5-trifluoromethoxyphenyl 4-(isobutanoylamino)benzoate, 2,5-dinitrophenyl 4-(isobutanoylamino)benzoate, 3-fluoro-6-nitrophenyl 4-(isobutanoylamino)benzoate, 3-chloro-6-nitrophenyl 4-(isobutanoylamino)benzoate, 3-methyl-6-nitrophenyl 4-(isobutanoylamino)benzoate, 2-nitro-5-(trifluoromethyl)phenyl 4-(isobutanoylamino)benzoate, 3-formyl-6-nitrophenyl 4-(isobutanoylamino)benzoate, 3-hydroxycarbonyl-6-nitrophenyl 4-(isobutanoylamino)benzoate, 3-aminocarbonyl-6-nitrophenyl 4-(isobutanoylamino)benzoate, 3-methoxycarbonyl-6-nitrophenyl 4-(isobutanoylamino)benzoate, 3-ethoxycarbonyl-6-nitrophenyl 4-(isobutanoylamino)benzoate, 3-(4-methoxybenzyloxy)-6-nitrophenyl 4-(isobutanoylamino)benzoate, 3-acetyl-6-nitrophenyl 4-(isobutanoylamino)-benzoate, 3-propanoyl-6-nitrophenyl 4-(isobutanoylamino)benzoate, 3-chloro-5-(cyclopropanecarbonyloxy)-phenyl 4-(pivaloylamino)benzoate, 3-chloro-5-(dimethylaminocarbonyloxy)phenyl 4-(pivaloylamino)benzoate, 3-chloro-5-(diethylaminocarbonyloxy)phenyl 4-(isobutanoylamino)benzoate, 3-fluoro-6-nitrophenyl 4-(pivaloylamino)benzoate, 3-formyl-6-nitrophenyl 4-(pivaloylamino)benzoate, 2-cyanophenyl 4-(isobutanoylamino)benzoate, 2-cyano-5-fluorophenyl 4-(isobutanoylamino)benzoate, 2-cyano-5-formylphenyl 4-(isobutanoylamino)benzoate, 2-cyano-5-hydroxyphenyl 4-(isobutanoylamino)benzoate, 2-cyano-5-hydroxycarbonylphenyl 4-(isobutanoylamino)benzoate, 2-cyano-5-methoxycarbonylphenyl 4-(isobutanoylamino)-benzoate, 2-cyano-5-trifluoromethoxyphenyl 4-(isobutanoylamino)benzoate, 3-acetoxy-6-cyanophenyl 4-(isobutanoylamino)benzoate, 2-cyano-5-isobutanoyloxyphenyl 4-(isobutanoylamino)benzoate, 2-cyano-5-(dimethylaminocarbonyloxy)phenyl 4-(isobutanoylamino)benzoate, 2-cyano-5-methylphenyl 4-(isobutanoylamino)benzoate, 2-cyano-5-trifluoromethylphenyl 4-(isobutanoylamino)benzoate, 2-cyano-5-nitrophenyl 4-(isobutanoylamino)benzoate, 3-acetyl-6-cyanophenyl 4-(isobutanoylamino)benzoate, 3-chloro-6-cyanophenyl 4-(isobutanoylamino)benzoate, 3-chloro-5-cyanophenyl 4-(isobutanoylamino)benzoate, 3-chloro-6-(methoxycarbonyl)phenyl 4-(isobutanoylamino)benzoate, 2,3,5-trichlorophenyl 4-(isobutanoylamino)-benzoate, 2,6-dichlorophenyl 4-(isobutanoylamino)benzoate, 2,3-difluorophenyl 4-(isobutanoylamino)-benzoate, 2,5-difluorophenyl 4-(isobutanoylamino)benzoate, 2,6-difluorophenyl 4-(isobutanoylamino)-benzoate, 3,5-difluorophenyl 4-(isobutanoylamino)benzoate, 3-chloro-5-fluorophenyl 4-(isobutanoylamino)-benzoate, 3-fluorophenyl 4-(isobutanoylamino)benzoate, 3-bromophenyl 4-(isobutanoylamino)benzoate, 3-chloro-5-hydroxyphenyl 4-(isobutanoylamino)benzoate, 3-acetoxy-5-chlorophenyl 4-(isobutanoylamino)-benzoate, 3-chloro-5-propanoyloxyphenyl 4-(isobutanoylamino)benzoate, 3-butanoyloxy-5-chlorophenyl 4-(isobutanoylamino)benzoate, 3-chloro-5-isobutanoyloxyphenyl 4-(isobutanoylamino)benzoate, 3-chloro-5-(cyclopropanecarbonyloxy)phenyl 4-(isobutanoylamino)benzoate, 3-chloro-5-(1-methylcyclopropanecar-bonyloxy)phenyl 4-(isobutanoylamino)benzoate, 3-chloro-5-(cyclobutanecarbonyloxy)phenyl 4-(isobutanoylamino)benzoate, 3-chloro-5-(cyclopentanecarbonyloxy)phenyl 4-(isobutanoylamino)benzoate, 3-chloro-5-(cyclohexanecarbonyloxy)phenyl 4-(isobutanoylamino)benzoate, 3-cinnamoyloxy-5-chlorophenyl 4-(isobutanoylamino)benzoate, 3-chloro-5-(dimethylaminocarbonyloxy)phenyl 4-(isobutanoylamino)benzoate, 3-chloro-5-(methanesulfonyloxy)phenyl 4-(isobutanoylamino)benzoate, 3-chloro-5-(ethanesulfonyloxy)phenyl 4-(isobutanoylamino)benzoate, 3-chloro-5-(phenylmethanesulfonyloxy)phenyl4-(isobutanoylamino)benzoate, 3-benzenesulfonyloxy-5-chlorophenyl 4-(isobutanoylamino)benzoate, 3-chloro-5-($\beta$-styrenesulfonyloxy)-phenyl 4-(isobutanoylamino)benzoate, 3-chloro-5-(2-thiophenesulfonyloxy)phenyl 4-(isobutanoylamino)-benzoate, 3-benzyloxy-5-chlorophenyl 4-(isobutanoylamino)benzoate, 3-fluoro-5-isobutanoyloxyphenyl 4-(isobutanoylamino)benzoate, 3-trifluoromethoxyphenyl 4-(isobutanoylamino)benzoate, 3-chloro-5-methoxyphenyl 4-(isobutanoylamino)benzoate, 3,5-dimethoxyphenyl 4-(isobutanoylamino)benzoate, 3-nitrophenyl 4-(isobutanoylamino)benzoate, 3-cyano-5-acetoxyphenyl 4-(isobutanoylamino)benzoate, 3-trifluoromethylphenyl 4-(isobutanoylamino)benzoate, 3,5-difluoromethyl 4-(isobutanoylamino)benzoate, 2,5-dinitrorophenyl 4-(meso-2,3-dimethylsuccinylamino)benzoate, 3-fluoro-6-nitrophenyl 4-(meso-2,3-dimethylsuccinylamino)-benzoate, 3-formyl-6-nitrophenyl 4-(meso-2,3-dimethylsuccinylamino)benzoate, 3-hydroxycarbonyl-6-nitrophenyl 4-(meso-2,3-dimethylsuccinylamino)benzoate, 3-methoxycarbonyl-6-nitrophenyl 4-(meso-2,3-dimethylsuccinylamino)benzoate, 3-chloro-5-(cyclopropanecarbonyloxy)phenyl 4-(meso-2,3-dimethylsuc-cinylamino)benzoate, 3-chloro-5-(dimethylaminocarbonyloxy)phenyl 4-(meso-2,3-dimethylsuccinylamino)-benzoate, 2-cyanophenyl 4-(meso-2,3-dimethylsuccinylamino)benzoate, 3-chloro-6-cyanophenyl 4-(meso-2,3-dimethylsuccinylamino)benzoate, 2,6-dichlorophenyl 4-(meso-2,3-dimethylsuccinylamino)benzoate, 3-ac-etoxy-5-chlorophenyl 4-(meso-2,3-dimethylsuccinylamino)benzoate, 3-chloro-5-propanoyloxyphenyl 4-(meso-2,3-dimethylsuccinylamino)benzoate, 3-chloro-5-isobutanoyloxyphenyl 4-(meso-2,3-dimethylsuc-cinylamino)benzoate, etc.

The compounds of formula [I] have potent inhibitory activity against human leukocyte elastase, which is a protease, and therefore are useful in the treatment and/or prevention of certain pathologic conditions associated with the leukocyte elastase activity, for example such diseases as pulmonary emphysema, arthritis, Crohn's disease and acute pancreatitis.

In administering the compounds of the invention to patients suffering from the diseases mentioned above as therapeutic agents for said diseases, the dose and route of administration are not critical but may vary depending on the kind of disease, severity of symptom, patient's age, condition and body weight, kind of concomitant treatment (if any), frequency of treatment, quality or extent of effect desired, and other factors. Generally, however, the compounds are administered at a daily dose of about 5 to 1,000 mg, preferably 10 to 500 mg, per human adult either orally or nonorally in a single does or divided doses.

As the dosage form, there may be mentioned, for example, powders, fine granules, granules, tablets, capsules, injections, suppositories, and nasal preparations. Such dosage forms may be produced by the conventional methods using the conventional carriers and other ingredients for pharmaceutical preparations.

Thus, for example, lactose, corn starch, white sugar, glucose, sorbitol, crystalline cellulose, silicon dioxide and the like may be used as excipients or diluents, polyvinyl alcohol, polyvinyl ether, ethylcellulose, methylcellulose, gum arabic, tragacanth gum, gelatin, shellac, hydroxypropylcellulose, hydroxypropylstarch, polyvinylpyrrolidone and the like as binders, starch, agar, pulverized gelatin, crystalline cellulose, calcium carbonate, sodium hydrogen carbonate, calcium citrate, calcium celluloseglycolate, dextrin, pectin and the like as disintegrating agents, magnesium sterate, talc, polyethylene glycol, silica, hardened vegetable oils and the like as lubricants, dyes and pigments approved as pharmaceuticals additives as coloring agents, and cocoa powder, peppermint oil, aromatic acids, powdered cinnamon bark and the like as corrigents or flavoring agents. The tablets and granules may of course be sugar-coated, gelatin-coated or coated with any other appropriate coating material.

For administration by injection, injectable compositions for intravenous administration can be prepared in the conventional manner by adding, for example, pH adjusting agents, buffering agents, stabilizers, solubilizers and other agents to the respective active ingreidnets.

The following examples are further illustrative of the present invention but are by no means limitative of the scope thereof. In the examples, nuclear magnetic resonance (NMR) spectrometry was performed on JNM FX-200, PMS-60SI and GX-400 model NMR spectrometers (mfd. by Nihon Denshi K.K.) with TMS as an internal standard and the shift data are expressed in terms of $\delta$ value (ppm).

EXAMPLE 1

Synthesis of 3,5-dichlorophenyl 4-(isobutanoylamino)benzoate (Compound 7):

A mixture of 28.5 g of 4-nitrobenzoyl chloride, 30.0 g of 3,5-dichlorophenol and 300 ml of pyridine was heated under reflux for 3 hours. After completion of the reaction, the pyridine was distilled off under reduced pressure, the remaining reaction product was dissolved in ethyl acetate, and the solution was washed with water and then with a saturated aqueous solution of sodium chloride and concentrated under reduced pressure to give crude crystals. Recrystallization of the crude crystals from ethyl acetate gave 44.2 g of 3,5-dichlorophenyl 4-nitrobenzoate as needles.

A 2.93-g portion of this compound was dissolved in 80 ml of isopropyl alcohol, a 10% palladium-on-carbon catalyst was added, and the mixture was stirred at room temperature for 5 hours in the presence of gaseous hydrogen (1 atmosphere). The catalyst was then filtered off and the solvent distilled off under reduced pressure, whereby 2.64 g of 3,5-dichlorophenyl 4-aminobenzoate was obtained as crystals.

A quantity of 3,5-Dichlorophenyl 4-aminobenzoate (282 mg) was dissolved in 5 ml of pyridine, 0.21 ml of isobutanoyl chloride was added to the solution with ice cooling, and the resulting mixture was stirred at room temperature for 10 hours. The pyridine was then distilled off under reduced pressure, water was added to the residue, and the mixture was extracted with ethyl acetate. The ethyl acetate layer was dried over sodium sulfate and then concentrated under reduced pressure, and the residue was recrystallized from hexane-ethyl acetate to give 168 mg of the title compound as needles.

EXAMPLE 2

Synthesis of 3,5-dichlorophenyl 4-(3-butylureido)benzoate (Compound 29):

A 5.64-g portion of the 3,5-dichlorophenyl 4-aminobenzoate obtained as described in Example 1 was dissolved in 100 ml of methylene chloride, 2.02 g of triethylamine and 1.98 g of triphosgene were added to the solution with ice cooling, and the resultant mixture was stirred at room temperature for 18 hours. The thus-produced 3,5-dichlorophenyl 4-isocyanatobenzoate was used in the next reaction step without purification. Thus, 5 ml of the reaction mixture containing said isocyanate was added to a mixture of 0.5 ml of butylamine and 5 ml of methylene chloride, and the resultnat mixture was stirred at room temperature for 30 minutes. The reaction mixture was washed with water, dried over sodium sulfate and then concentrted under reduced pressure. The residual solid was recrystallized from chloroform to give 159 mg of the title compound as white crystals.

EXAMPLE 3

Synthesis of 3-formyl-6-nitrophenyl 4-(pivaloylamino)benzoate(Compound 60):

4-Aminobenzoic acid (6.86 g) was suspended in 300 ml of diethyl ether. Triethylamine (13.9 ml) and 15.4 ml of trimethylacetyl chloride were added to the suspension, and the mixture was stirred at room temperature for 13 hours. After completion of the reaction, the solid was filtered off and washed with diethyl ether. The filtrate and the washings were combined and washed with water. The ether layer was concentrated under reduced pressure and the crude crystals thus obtained were recrystallized from ethanol-toluene to give 7.97 g of 4-(pivaloylamino)benzoic acid.

A 0.7-g portion of this compound was dissolved in 30 ml of pyridine, and 1.05 g of 3-formyl-6-nitrophenol, 1.88 g of dicyclohexylcarbodiimide and 0.45 g of 4-dimethylaminopyridine were added to the solution. The mixture was stirred at room temperature for 15 hours. The resultant solid was filtered off, and the filtrate was concentrated under reduced pressure. The concentrate was purified by silica gel column chromatography (developing solvent: toluene-ethyl acetate = 10:1) to give 0.67 g of the title compound as crystals.

The nuclear magnetic resonance spectrum data for Compounds 1 to 94, which are typical compounds of the present invention synthesized according to any method of Examples 1 to 3, are shown in Table 1

(DMSO-d6 was used unless otherwise indicated).

Table 1

| compound | R1 | R2 | R3 | R4 | R5 | R6 | NMR data |
|---|---|---|---|---|---|---|---|
| 1 | 1-Methylcyclo-propanecarbonyl | H | H | Cl | H | Cl | 0.57-0.83(m, 2H), 1.24-1.43(m, 2H), 1.45(s, 1H), 7.00(s, 2H), 7.03(s, 1H), 7.60(d, 9Hz, 2H), 7.61(s, 1H), 7.95(d, 9Hz, 2H) ($CDCl_3$) |
| 2 | Propanoyl | H | H | Cl | H | Cl | 1.10(t, 8Hz, 3H), 2.40(q, 8Hz, 2H), 7.52(s, 2H) 7.57(s, 1H), 7.82(d, 9Hz, 2H), 8.06(d, 9Hz, 2H) 10.34(s, 1H) |
| 3 | Butanoyl | H | H | Cl | H | Cl | 0.95(t, 8Hz, 3H), 1.59-1.70(m, 2H), 2.36(t, 8Hz, 2H), 7.52(s, 2H), 7.60(s, 1H), 7.84(d, 8Hz 2H), 8.06(d, 8Hz, 2H), 10.34(s, 1H) |
| 4 | Pentanoyl | H | H | Cl | H | Cl | 0.91(t, 7Hz, 3H), 1.33(d of t, 7Hz, 2H), 1.60(d of t, 7Hz, 2H), 2.34(t, 7Hz, 2H), 7.53(s, 2H), 7.56(s, 1H), 7.81(d, 9Hz, 2H), 8.06(d, 9Hz, 2H), 10.33(s, 1H) |
| 5 | Hexanoyl | H | H | Cl | H | Cl | 0.89(t, 8Hz, 3H), 1.27-1.34(m, 4H), 1.58-1.63(m, 2H), 2.31-2.39(m, 2H), 7.53(s, 2H), 7.59(s, 1H), 7.82(d, 8Hz, 2H), 8.06(d, 8Hz, 2H), 10.37(s, 1H) |
| 6 | Octanoyl | H | H | Cl | H | Cl | 0.86(t, 7Hz, 3H), 1.27-1.30(m, 8H), 1.61(t, 7Hz, 2H), 7.52(s, 2H), 7.59(s, 1H), 7.84(d, 8Hz, 2H), 8.05(d, 8Hz, 2H), 10.33(s, 1H) |
| 7 | Isobutanoyl | H | H | Cl | H | Cl | 1.13(d, 6Hz, 6H), 2.65(sept, 6Hz, 1H), 7.51 (d, 1Hz, 2H), 7.56(t, 1Hz, 1H), 7.84(d, 9Hz, 2H) 8.06(d, 9Hz, 2H), 10.29(s, 1H) |
| 8 | Pivaloyl | H | H | Cl | H | Cl | 1.26(s, 9H), 7.53(s, 2H), 7.58(s, 1H), 7.91(d, 9Hz, 2H), 8.07(d, 9Hz, 2H), 9.63(s, 1H) |

Table 1(cont'd)

| compound | R1 | R2 | R3 | R4 | R5 | R6 | NMR data |
|---|---|---|---|---|---|---|---|
| 9 | Phenylacetyl | H | H | Cl | H | Cl | 3.72(s, 2H), 7.25-7.39(m, 5H), 7.53(s, 2H), 7.59(s, 1H), 7.82(d, 9Hz, 2H), 8.07(d, 9Hz, 2H) 10.63(s, 1H) |
| 10 | Trichloro -acetyl | H | H | Cl | H | Cl | 7.19(s, 2H), 7.29(s, 1H), 7.67(d, 9Hz, 2H), 8.22(d, 9Hz, 2H), 8.52(s, 1H) ($CDCl_3$) |
| 11 | Trifluoro -acetyl | H | H | Cl | H | Cl | 7.15(d, 1Hz, 2H), 7.27(t, 1Hz, 1H), 7.77(d, 10Hz, 2H), 8.18(s, 1H), 8.19(d, 10Hz, 2H) ($CDCl_3$) |
| 12 | Pentafluoro -propanoyl | H | H | Cl | H | Cl | 7.18(d, 1Hz, 2H), 7.27(t, 1Hz, 1H), 7.74(d, 9Hz, 2H), 8.20(d, 9Hz, 2H), 8.25(s, 1H) ($CDCl_3$) |
| 13 | 2-Butenoyl | H | H | Cl | H | Cl | 1.90(d, 7Hz, 3H), 6.17(d, 15Hz, 1H), 6.88(d of q, d;15Hz, q;7Hz, 1H), 7.53(s, 2H), 7.59(s, 1H) 7.87(d, 9Hz, 2H), 8.08(d, 9Hz, 2H), 10.4(s, 1H) |
| 14 | 2-Methyl -propenoyl | H | H | Cl | H | Cl | 1.98(s, 3H), 5.61(s, 1H), 5.89(s, 1H), 7.53(s, 2H), 7.58(s, 1H); 7.94(d, 9Hz, 2H), 8.08(d, 9Hz 2H), 10.21(s, 1H) |
| 15 | Cyclopropane carbonyl | H | H | Cl | H | Cl | 0.86(d, 6Hz, 4H), 1.82-1.88(m, 1H), 7.53(s, 2H), 7.59(s, 1H), 7.81(d, 8Hz, 2H), 8.07(d, 8Hz, 2H), 10.71(s, 1H) |
| 16 | Cyclobutane carbonyl | H | H | Cl | H | Cl | 1.78-2.00(m, 2H), 2.10-2.29(m, 4H), 3.22-3.37(m, 1H), 7.53(s, 2H), 7.59(s, 1H), 7.81(d, 8Hz, 2H), 8.06(d, 8Hz, 2H) |

Table 1 (cont'd)

| compound | R1 | R2 | R3 | R4 | R5 | R6 | NMR data |
|---|---|---|---|---|---|---|---|
| 17 | Cyclopentane carbonyl | H | H | Cl | H | Cl | 1.52-1.93(m, 8H), 2.84(m, 1H), 7.53(s, 2H), 7.59(s, 1H), 7.83(d, 9Hz, 2H), 8.06(d, 9Hz, 2H), 10.32(s, 1H) |
| 18 | Cyclohexane carbonyl | H | H | Cl | H | Cl | 1.15-1.48(m, 5H), 1.60-1.82(m, 5H), 2.35-2.42(m, 1H), 7.47(s, 2H), 7.57(s, 1H), 7.80(d, 9Hz, 2H), 8.03(d, 9Hz, 2H), 10.31(s, 1H) |
| 19 | 3-Methyl-butanoyl | H | H | Cl | H | Cl | 1.03(d, 5Hz, 6H), 1.10-1.33(m, 1H), 2.27(d, 2Hz, 2H), 7.14(d, 1Hz, 1H), 7.24(t, 1Hz, 1H), 7.67(d, 6Hz, 2H), 7.73(s, 1H), 8.10(d, 6Hz, 2H) ($CDCl_3$) |
| 20 | 2-Methyl-2-phenyl acetyl | H | H | Cl | H | Cl | 1.62(d, 7Hz, 3H), 3.70(q, 7Hz, 1H), 7.00-8.03(m, 8H), 7.36(d, 9Hz, 2H), 7.48(s, 1H), 7.87(d, 9Hz, 2H) ($CDCl_3$) |
| 21 | Cinnamoyl | H | H | Cl | H | Cl | 6.69(d, 15Hz, 1H), 7.42(d, 15Hz, 1H), 7.15-7.74(m, 8H), 7.90(d, 9Hz, 2H), 7.99(s, 1H), 8.26(d, 9Hz, 2H) ($CDCl_3$) |
| 22 | Succinyl | H | H | Cl | H | Cl | 2.51-2.66(m, 4H); 7.52(s, 2H), 7.58(s, 1H), 7.81(d, 9Hz, 2H), 8.06(d, 9Hz, 2H), 10.44(s, 1H) |
| 23 | $-CO-CH_2CH_2-CO-$ | | H | Cl | H | Cl | 2.80-2.84(m, 4H), 7.55(d, 9Hz, 2H), 7.56(s, 2H), 7.61(s, 1H), 8.23(d, 9Hz, 2H) |
| 24 | Methane-sulfonyl | H | H | Cl | H | Cl | 3.16(s, 3H), 7.35(d, 8Hz, 2H), 7.52(s, 2H), 7.59(s, 1H), 8.06(d, 8Hz, 2H), 10.52(s, 1H) |

Table 1 (cont'd)

| compound | R1 | R2 | R3 | R4 | R5 | R6 | NMR data |
|---|---|---|---|---|---|---|---|
| 25 | Benzene -sulfonyl | H | H | Cl | H | Cl | 7.31(d, 9Hz, 2H), 7.48(s, 2H), 7.53-7.68(m, 4H), 7.87(d, 8Hz, 2H), 8.00(d, 8Hz, 2H), 11.06(s, 1H) |
| 26 | N-Methyl -amino -carbonyl | H | H | Cl | H | Cl | 6.24(s, 1H), 7.50(d, 1Hz, 2H), 7.59(s, 1H), 7.60(d, 9Hz, 2H), 7.98(d, 9Hz, 2H), 9.45(s, 1H) |
| 27 | N-Ethyl -amino -carbonyl | H | H | Cl | H | Cl | 1.09(t, 8Hz, 3H), 3.10-3.19(m, 2H), 6.34(t, 6Hz, 1H), 7.50(s, 2H), 7.57(s, 1H), 7.60(d, 9Hz, 2H), 7.98(d, 9Hz, 2H), 9.02(s, 1H) |
| 28 | N-Propyl -amino -carbonyl | H | H | Cl | H | Cl | 0.88(t, 8Hz, 3H), 1.40-1.54(m, 2H), 3.05-3.10(m, 2H), 6.38(t, 6Hz, 1H), 7.50(s, 2H), 7.58(s, 1H), 7.60 &7.98 each(d, 9Hz, 2H), 9.01(s, 1H) |
| 29 | N-Butyl -amino -carbonyl | H | H | Cl | H | Cl | 0.90(t, 8Hz, 3H), 1.27-1.36(m, 2H), 1.41-1.50(m, 2H), 3.10(t, 6Hz, 2H), 6.34(t, 6Hz, 1H), 7.50(s, 2H), 7.58(s, 1H), 7.60(d, 9Hz, 2H), 8.00(d, 9Hz, 2H), 8.99(s, 1H) |
| 30 | N-Pentyl -amino -carbonyl | H | H | Cl | H | Cl | 0.89(t, 7Hz, 3H), 1.23-1.36(m, 4H), 1.39-1.49(m, 2H), 3.10(q, 7Hz, 2H), 6.36(t, 6Hz, 1H), 7.51(s, 2H), 7.58(s, 1H), 7.60(d, 9Hz, 2H), 7.98(d, 9Hz, 2H), 8.99(s, 1H) |
| 31 | N-Isopropyl -amino -carbonyl | H | H | Cl | H | Cl | 1.11(d, 6Hz, 6H), 3.78(d of q, d;8Hz, q;6Hz, 1H), 6.25(d, 8Hz, 1H), 7.51(s, 2H), 7.58(s, 1H) 7.60(d, 9Hz, 2H), 8.00(d, 9Hz, 2H), 8.89(s, 1H) |
| 32 | N,N-Dimchtyl -amino -carbonyl | H | H | Cl | H | Cl | 2.96(s, 6H), 7.51(s, 2H), 7.58(s, 1H), 7.73(d, 9Hz, 2H), 7.99(d, 9Hz, 2H), 8.81(s, 1H) |

Table 1 (cont'd)

| compound | R1 | R2 | R3 | R4 | R5 | R6 | NMR data |
|---|---|---|---|---|---|---|---|
| 33 | Methoxy -carbonyl | H | H | Cl | H | Cl | 3.72(s, 3H), 7.52(s, 2H), 7.57(s, 1H), 7.67(d, 9Hz, 2H), 8.05(d, 9Hz, 2H), 10.20(s, 1H) |
| 34 | Benzyloxy -carbonyl | H | H | Cl | H | Cl | 5.27(s, 2H), 6.73(d, 2Hz, 2H), 6.92(t, 2Hz, 1H) 7.00(s, 1H), 7.35-7.42(m, 5H), 7.53(d, 9Hz, 2H), 8.10(d, 9Hz, 2H) ($CDCl_3$) |
| 35 | (2-Hydroxy -ethoxy) -carbonyl | H | H | Cl | H | Cl | 3.63-3.69(m, 2H), 4.15-4.21(m, 2H), 4.86(t, 5Hz, 1H), 7.50(s, 2H), 7.57(s, 1H), 7.71(d, 9Hz, 2H), 8.04(d, 9Hz, 2H), 10.26(s, 1H) |
| 36 | Acetoxy -carbonyl | H | H | Cl | H | Cl | 2.11(s, 3H), 7.51(s, 2H), 7.57(s, 1H), 7.80(d, 9Hz, 2H), 8.06(d, 9Hz, 2H), 10.42(s, 1H) |
| 37 | 2-Thenyl -acetyl | H | H | Cl | H | Cl | 3.97(s, 2H), 7.05-7.40(m, 7H), 7.59(d, 6Hz, 2H), 8.09(d, 6Hz, 2H) ($CDCl_3$) |
| 38 | 2-Methylthio propanoyl | H | H | Cl | H | Cl | 1.59(d, 8Hz, 3H), 2.17(s, 3H), 3.50(q, 8Hz, 1H), 7.18(d, 1Hz, 2H), 7.27(d, 1Hz, 1H), 7.74(d, 9Hz, 2H), 8.14(d, 9Hz, 2H), 8.84(s, 1H) |
| 39 | 2-Mercapto -propanoyl | H | H | Cl | H | Cl | 1.52(d, 7Hz, 3H), 3.53(s, 1H), 3.25(q, 7Hz, 1H), 7.15(d, 2Hz, 2H), 7.26(t, 2Hz, 1H), 7.83(d, 9Hz, 2H), 8.18(d, 9Hz, 2H), 8.30(s, 1H) ($CDCl_3$) |
| 40 | 2,2-Dimethyl -malonyl | H | H | Cl | H | Cl | 1.13(s, 6H), 7.46(s, 3H), 7.76(d, 9Hz, 2H), 8.03(d, 9Hz, 2H), 10.16(s, 1H) |

Table 1 (cont'd)

| compound | R1 | R2 | R3 | R4 | R5 | R6 | NMR data |
|---|---|---|---|---|---|---|---|
| 41 | 3,3-Dimethyl-succinyl | H | H | Cl | H | Cl | 1.23(s, 6H), 2.68(s, 2H), 7.52(s, 2H), 7.58(s, 1H), 7.82(d, 9Hz, 2H), 8.08(d, 9Hz, 2H), 10.35(s, 1H), 12.50(s, 1H) |
| 42 | meso-2,3-Dimethyl-succinyl | H | H | Cl | H | Cl | 1.05(d, 3Hz, 3H), 1.15(d, 2Hz, 3H), 2.36-2.72(m, 2H), 7.36(s, 3H), 7.67(d, 9Hz, 2H), 7.96(d, 9Hz, 2H), 10.25(s, 1H) ($CD_3OD$) |
| 43 | Glutaryl | H | H | Cl | H | Cl | 1.12-1.42(m, 2H), 1.82-2.19(m, 2H), 2.28-2.64(m, 2H), 7.23(d, 2Hz, 2H), 7.31(d, 2Hz, 1H), 7.63(s, 1H), 7.78(d, 9Hz, 2H), 8.12(d, 9Hz, 2H) ($CD_3OD$) |
| 44 | (2-Hydroxycarb-onyl)cyclohex-anecarbonyl | H | H | Cl | H | Cl | 1.24-2.17(m, 8H), 2.62-2.95(m, 2H), 7.12-7.56(m, 3H), 7.72(d, 9Hz, 2H), 8.07(d, 9Hz, 2H) |
| 45 | 3-Chloro-2,2-dimethyl-propanoyl | H | H | Cl | H | Cl | 1.35(s, 6H), 3.85(s, 2H), 7.42(s, 3H), 7.76(d, 9Hz, 2H), 8.03(d, 9Hz, 2H), 10.16(s, 1H) |
| 46 | 3-Cyano-2,2-dimethyl-propanoyl | H | H | Cl | H | Cl | 1.33(s, 6H), 3.56(s, 2H), 7.43(d, 9Hz, 2H), 7.47(s, 3H), 8.07(d, 9Hz, 2H) |
| 47 | tert-Butoxy-carbonyl | H | $NO_2$ | H | H | CHO | 1.53(s, 9H), 7.66(d, 9Hz, 2H), 7.87-8.40(m, 3H), 8.03(d, 9Hz, 2H), 9.87(s, 1H), 10.07(s, 1H) |
| 48 | Isobutanoyl | H | $NO_2$ | H | H | H | 1.12(d, 6Hz, 6H), 2.57(sept, 6Hz, 1H), 7.40-8.26(m, 3H), 7.82(d, 9Hz, 2H), 8.08(d, 9Hz, 2H), 10.26(s, 1H) |

Table 1 (cont'd)

| compound | R1 | R2 | R3 | R4 | R5 | R6 | NMR data |
|---|---|---|---|---|---|---|---|
| 49 | Isobutanoyl | H | $-NO_2$ | $-OH$ | H | H | 1.12(d, 6Hz, 6H), 2.22-2.88(m, 2H), 6.83-7.61(m, 3H), 7.74(d, 9Hz, 2H), 7.96(d, 9Hz, 2H), 10.18(d, 1Hz, 1H) |
| 50 | Isobutanoyl | H | $-NO_2$ | H | H | $-NO_2$ | 1.29(d, 6Hz, 6H), 2.59(sept, 6Hz, 1H), 7.74(d, 9Hz, 2H), 8.13(d, 9Hz, 2H), 8.23-8.33(m, 3H) $(CDCl_3)$ |
| 51 | Isobutanoyl | H | $-NO_2$ | H | H | F | 1.22(d, 6Hz, 6H), 2.25-2.65(m, 1H), 6.66-7.20(m, 3H), 7.50(d, 8Hz, 2H), 7.91(d, 8Hz, 2H), 7.97(s, 1H) $(CDCl_3)$ |
| 52 | Isobutanoyl | H | $-NO_2$ | H | H | $-CH_3$ | 1.24(d, 7Hz, 6H), 2.39-2.77(m, 1H), 2.56(s, 3H), 7.16-7.26(m, 2H), 7.68(d, 10Hz, 2H), 8.03(d, 10Hz, 2H), 8.11(d, 10Hz, 2H) $(CDCl_3)$ |
| 53 | Isobutanoyl | H | $-NO_2$ | H | H | $-CHO$ | 1.12(d, 7Hz, 2H), 2.20-2.90(m, 1H), 7.83(d, 9Hz, 2H), 7.86-8.43(m, 5H), 8.10(d, 9Hz, 2H), 10.10(s, 1H), 10.27(s, 1H) |
| 54 | Isobutanoyl | H | $-NO_2$ | H | H | $-COOH$ | 1.13(d, 6Hz, 6H), 2.25-2.92(m, 1H), 7.79(d, 9Hz, 2H), 7.95-8.29(m, 5H), 10.2(s, 1H) |
| 55 | Isobutanoyl | H | $-NO_2$ | H | H | $-COOCH_3$ | 1.26(d, 7Hz, 7H), 2.60(sept, 7Hz, 1), 3.97(s, 3H), 7.75(d, 10Hz, 2H), 7.98-8.13(m, 4H), 8.14(d, 10Hz, 2H) $(CDCl_3)$ |
| 56 | Isobutanoyl | H | $-NO_2$ | H | H | (4-Methoxy -benzyloxy) -carbonyl | 1.13(d, 6Hz, 6H), 2.30-3.04(m, 1H), 3.73(s, 3H), 5.31(s, 2H), 6.89(d, 8Hz, 2H), 7.38(d, 8Hz2H), 7.74-8.33(m, 2H), 7.81(d, 8Hz, 2H), 8.04(d, 8Hz, 2H), 10.23(s, 1H) |

Table 1 (cont'd)

| compound | R1 | R2 | R3 | R4 | R5 | R6 | NMR data |
|---|---|---|---|---|---|---|---|
| 57 | Pivaloyl | H | H | Cyclopropane -carbonyloxy | H | Cl | 0.92-1.21(m, 4H), 1.36(s, 9H), 1.60-2.07(m, 1H), 6.89-7.15(m, 3H), 7.58(s, 1H), 7.68(d, 9Hz, 2H), 8.11(d, 9Hz, 2H) ($CDCl_3$) |
| 58 | Pivaloyl | H | H | N,N-Dimethylaminocarbonyloxy | H | Cl | 1.32(s, 9H), 3.04(s, 3H), 3.10(s, 3H), 6.97-7.21(m, 3H), 7.74(d, 9Hz, 2H), 8.01(s, 1H), 8.16(d, 9Hz, 2H) ($CDCl_3$) |
| 59 | Pivaloyl | H | $-NO_2$ | H | H | F | 1.26(s, 9H), 7.37(s, 1H), 7.47-7.57(m, 1H), 7.71-7.74(m, 1H), 7.94(d, 9Hz, 2H), 8.08(d, 9Hz, 2H), 8.33(dd, 10Hz, 6Hz, 1H), 9.66(s, 1H) |
| 60 | Pivaloyl | H | $-NO_2$ | H | H | -CHO | 1.34(s, 9H), 7.56-7.97(m, 4H), 8.02(d, 5Hz, 2H), 8.21(d, 5Hz, 2H), 10.10(s, 1H) ($CDCl_3$) |
| 61 | Isobutanoyl | H | -CN | H | H | H | 1.13(d, 7Hz, 6H), 2.33-2.80(m, 1H), 7.16-7.70(m, 4H), 7.70(d, 8Hz, 2H), 7.96(d, 8Hz, 2H) |
| 62 | Isobutanoyl | H | -CN | H | H | Cl | 1.13(d, 7Hz, 6H), 2.47-2.86(m, 1H), 7.15-8.05(m, 4H), 7.89(d, 8Hz, 2H), 8.14(d, 8Hz, 2H) |
| 63 | Isobutanoyl | H | $-COOCH_3$ | H | H | Cl | 1.23(d, 7Hz, 6H), 2.15-2.83(m, 1H), 3.71(s, 1H), 7.10-7.86(m, 3), 7.54(d, 9Hz, 2H), 7.99(d, 9Hz, 2H), 8.18(s, 1H) ($CDCl_3$) |
| 64 | Isobutanoyl | H | H | $-CF_3$ | H | $-CF_3$ | 1.27(d, 7Hz, 6H), 2.56(sept, 7Hz, 1H), 7.60-7.77(m, 3H), 7.70(d, 9Hz, 2H), 8.12(d, 9Hz, 2H) ($CDCl_3$) |

Table 1 (cont'd)

| compound | R1 | R2 | R3 | R4 | R5 | R6 | NMR data |
|---|---|---|---|---|---|---|---|
| 65 | Isobutanoyl | H | Cl | Cl | H | Cl | 1.15(d, 7Hz, 6H), 2.67(sept, 6Hz, 1H), 7.76(d, 2Hz, 1H), 7.85(d, 2Hz, 1H), 7.88(d, 9Hz, 2H), 8.17(d, 9Hz, 2H), 10.33(s, 1H) |
| 66 | Isobutanoyl | H | Cl | H | Cl | H | 1.22(d, 6Hz, 6H), 2.53(sept, 6Hz, 1H), 6.96-7.46(m, 3H), 7.69(d, 9Hz, 2H), 7.87(s, 1H), 8.19(d, 9Hz, 2H) ($CDCl_3$) |
| 67 | Isobutanoyl | H | F | F | H | H | 1.30(d, 6Hz6H), 2.57(sept, 6Hz, 1H), 7.02-7.13(m, 3H), 7.35(s, 1H), 7.71(d, 9Hz, 2H), 8.18(d, 9Hz, 2H) ($CDCl_3$) |
| 68 | Isobutanoyl | H | F | H | H | F | 1.27(d, 6Hz, 6H), 2.57(sept, 6Hz, 1H), 7.01-7.27(m, 3H), 7.73(d, 9Hz, 2H), 7.76(s, 1H), 8.20(d, 9Hz, 2H) ($CDCl_3$) |
| 69 | Isobutanoyl | H | F | H | F | H | 1.27(d, 6Hz, 6H), 2.56(sept, 6Hz, 1H), 7.01(t, 10Hz, 2H), 7.16-7.23(m, 1H), 7.51(s, 1H), 7.72(d, 9Hz, 2H), 8.18(d, 9Hz, 2H) ($CDCl_3$) |
| 70 | Isobutanoyl | H | H | F | H | F | 1.22(d, 6Hz, 6H), 2.55(sept, 6Hz, 1H), 6.60-6.86(m, 3H), 7.71(d, 9Hz, 2H), 7.84(s, 1H), 8.11(d, 9Hz, 2H) ($CDCl_3$) |
| 71 | Isobutanoyl | H | H | F | H | H | 1.27(d, 6Hz, 6H), 2.50(sept, 6Hz, 1H), 6.78-7.42(m, 4H), 7.68(d, 8Hz, 2H), 7.80(s, 1H), 8.08(d, 8Hz, 2H) ($CDCl_3$) |
| 72 | Isobutanoyl | H | H | Br | H | H | 1.27(d, 7Hz, 6H), 2.56(sept, 7Hz, 1H), 7.08-7.56(m, 4H), 7.76(d, 9Hz, 2H), 8.10(s, 1H), 8.17(d, 9Hz, 2H) |

Table 1 (cont'd)

| compound | R1 | R2 | R3 | R4 | R5 | R6 | NMR data |
|---|---|---|---|---|---|---|---|
| 73 | Isobutanoyl | H | H | -OH | H | Cl | 1.13(d, 6Hz, 6H), 2.54(sept, 6Hz, 1H), 6.60-6.87(m, 3H), 7.87(d, 9Hz, 2H), 8.11(d, 9Hz, 2H) |
| 74 | Isobutanoyl | H | H | Acetoxy | H | Cl | 1.11(d, 6Hz, 6H), 2.15-2.89(m, 1H), 7.11-7.44(m, 3H), 7.81(d, 9Hz, 2H), 8.07(d, 9Hz, 2H), 10.22(s, 1H) |
| 75 | Isobutanoyl | H | H | Propanoyloxy | H | Cl | 1.20(d, 6Hz, 6H), 1.22(t, 7Hz, 3H), 2.21-2.80(m, 3H), 6.73-7.10(m, 3H), 7.46(d, 8Hz, 2H), 7.78(s, 1H), 7.86(d, 8Hz, 2H) ($CDCl_3$) |
| 76 | Isobutanoyl | H | H | Isobutanoyloxy | H | Cl | 1.12(d, 6Hz, 6H), 1.24(d, 6Hz, 6H), 2.20-2.92(m, 2H), 7.10-7.39(m, 3H), 7.75(d, 9Hz, 2H), 8.01(d, 9Hz, 2H), 10.17(s, 1H) |
| 77 | Isobutanoyl | H | H | Cyclopropane -carbonyloxy | H | Cl | 0.92-1.25(m, 4H), 1.23(d, 6Hz, 6H), 1.66-2.03(m, 1H), 2.53(sept, 7Hz, 1H), 6.97(t, 2Hz, 1H), , 7.05(t, 2Hz, 1H), 7.12(t, 2Hz, 1H), 7.57(d, 9Hz, 2H), 7.95(s, 1H), 8.08(d, 9Hz, 2H) ($CDCl_3$) |
| 78 | Isobutanoyl | H | H | 1-Methyl -cyclopropane -carbonyloxy | H | Cl | 0.72-0.98(m, 4H), 1.20(d, 6Hz, 6H), 1.35(s, 3H), 1.98-2.66(m, 1H), 6.70-7.10(m, 3H), 7.46(d 9Hz, 2H), 7.90(d, 9Hz, 2H), 7.95(s, 1H) ($CDCl_3$) |
| 79 | Isobutanoyl | H | H | Cyclohexane -carbonyloxy | H | Cl | 1.22(d, 7Hz, 6H), 1.33-2.20(m, 11H), 2.18-2.82(m, 1H), 6.73-7.03(m, 3H), 7.48(d, 9Hz, 2H), 7.90(d, 9Hz, 2H), 8.00(s, 1H) ($CDCl_3$) |
| 80 | Isobutanoyl | H | H | Cinnamoyloxy | H | Cl | 1.22(d, 7Hz, 6H), 2.33-2.63(m, 1H), 6.23-7.73(m, 10H), 7.47(d, 9Hz, 2H), 7.58(s, 1H), 7.91(d, 9Hz, 2H) ($CDCl_3$) |

Table 1(cont'd)

| compound | R1 | R2 | R3 | R4 | R5 | R6 | NMR data |
|---|---|---|---|---|---|---|---|
| 81 | Isobutanoyl | H | H | N,N-Dimethyl -amino -carbonyloxy | H | Cl | 1.20(d,7Hz,6H),2.52(sept,7Hz,1H),3.06(d,7Hz,6H),6.97-7.20(m,3H),7.72(d,9Hz,2H),8.07(d,9Hz,2H),8.78(s,1H) ($CDCl_3$) |
| 82 | Isobutanoyl | H | H | Methane -sulfonyloxy | H | Cl | 1.24(d,7Hz,6H),2.57(sept,7Hz,1H),3.22(s,3H),7.06-7.38(m,3H),7.63(s,1H),7.76(d,9Hz,2H),8.18(d,9Hz,2H) ($CDCl_3$) |
| 83 | Isobutanoyl | H | H | Ethane -sulfonyloxy | H | Cl | 1.27(d,7Hz,6H),1.53(t,7Hz,3H),2.26-2.68(m,1H),3.65(q,7Hz,2H),6.83-7.30(m,3H),7.51(d,9Hz,2H),7.53(s,1H),7.93(d,9Hz,2H) ($CDCl_3$) |
| 84 | Isobutanoyl | H | H | Phenyl -methane -sulfonyloxy | H | Cl | 1.23(d,7Hz,6H),2.20-2.63(m,1H),4.86(s,2H),6.73-7.23(m,2H),7.26(s,5H),7.44(s,1H),7.50(d,9Hz,2H),7.90(d,9Hz,2H) ($CDCl_3$) |
| 85 | Isobutanoyl | H | H | Benzene -sulfonyloxy | H | Cl | 1.24(d,7Hz,6H),2.56(sept,7Hz,1H),6.66-7.75(m,8H),7.56(d,9Hz,2H),7.87(d,9Hz,2H),8.50(s,1H) ($CDCl_3$) |
| 86 | Isobutanoyl | H | H | β-Styrene -sulfonyloxy | H | Cl | 1.28(d,7Hz,6H),2.50(sept,7Hz,1H),6.87(d,17Hz,1H),7.19(m,3H),7.27(s,5H),7.63(d,17Hz,1H),7.71(d,9Hz,2H),7.77(s,1H),8.07(d,9Hz,2H) ($CDCl_3$) |
| 87 | Isobutanoyl | H | H | 2-Thiophene -sulfonyloxy | H | Cl | 1.31(d,7Hz,6H),2.62(sept,7Hz,1H),6.76(m,4H),7.60(d,8Hz,2H),7.47-7.65(m,2H),7.94(d,8HZ,2H),7.96(s,1H) ($CDCl_3$) |
| 88 | Isobutanoyl | H | H | Benzyloxy | H | Cl | 1.21(d,7Hz,6H),2.20-2.83(m,1H),4.90(s,2H),6.53-6.76(m,3H),7.18(s,5H),7.36(d,9Hz,2H),7.55(s,1H),7.91(d,9Hz,2H) ($CDCl_3$) |

Table 1 (cont'd)

| compound | R1 | R2 | R3 | R4 | R5 | R6 | NMR data |
|---|---|---|---|---|---|---|---|
| 89 | Isobutanoyl | H | H | $-OCF_3$ | H | H | 1.26(d, 7Hz, 6H), 2.56(sept, 7Hz, 1H), 7.00-7.53(m, 4H), 7.69(d, 9Hz, 2H), 7.74(s, 1H), 8.13(d, 9Hz, 2H) ($CDCl_3$) |
| 90 | Isobutanoyl | H | H | $-OCH_3$ | H | Cl | 1.30(d, 7Hz, 6H), 2.56(sept, 7Hz, 1H), 3.82(s, 3H), 6.68(t, 2Hz, 1H), 6.75-6.91(m, 2H), 7.48(s, 1H), 7.68(d, 9Hz, 2H), 8.13(d, 9Hz, 2H) ($CDCl_3$) |
| 91 | Isobutanoyl | H | H | $-OCH_3$ | H | $-OCH_3$ | 1.14(d, 6Hz, 6H), 2.66(sept, 6Hz, 1H), 3.77(s, 6H), 6.45(s, 1H), 6.47(s, 2H), 7.83(d, 10Hz, 2H), 8.06(d, 10Hz, 2H) |
| 92 | Isobutanoyl | H | H | $-NO_2$ | H | H | 1.15(d, 6Hz, 6H), 2.45(sept, 6Hz, 1H), 6.95-7.20(m, 5H), 7.50(d, 8Hz, 2H), 7.60(s, 1H), 7.95(d, 8Hz, 2H) ($CDCl_3$) |
| 93 | Isobutanoyl | H | H | $-CN$ | H | Acetoxy | 1.29(d, 7Hz, 6H), 2.26(s, 3), 2.57(sept, 7Hz, 1H), 7.34-7.49(m, 3H), 7.71(d, 10Hz, 2H), 8.12(d, 10Hz, 2H) ($CDCl_3$) |
| 94 | Isobutanoyl | H | H | $-CF_3$ | H | H | 1.20(d, 7Hz, 6H), 2.43-2.83(m, 1H), 7.49-7.64(m, 3H), 7.84(d, 9Hz, 2H), 7.87(s, 1H), 8.12(d, 9Hz, 2H), 9.98(s, 1H) ($CDCl_3$-$DMSOd_6$) |

DOSAGE FORM EXAMPLE 1

According to the established pharmaceutical procedures, a mixture of 10 g of 3,5-dichlorophenyl 4-(isobutanoylamino)benzoate (Compound 7), 400 mg of calcium celluloseglycolate (disintegrating agent), 200

mg of magnesium stearate (lubricant) and 9.4 g of crystalline cellulose was tabletted to give 100 tablets each containing 100 mg of the active ingredient.

DOSAGE FORM EXAMPLE 2

A quantity of 3,5-Dichlorophenyl 4-(2-hydroxyethoxyamino)benzoate (Compound 50) (1 g) was dissolved in 10 ml of ethanol, and the solution was sterilized by it passing through a bacterial filter and then distributed in 0.5-ml portions into 5-ml ampules, which were then sealed. Each ampule contained 50 mg of the active ingredient. For injection, the ampule contents are to be diluted with an appropriate volume of an appropriate diluent, for example 2.5 ml of Tris-hydrochloride buffer (pH 8.6).

The following test example illustrates some typical pharmacological properties of the compounds of the present invention.

PHARMACOLGICAL TEST EXAMPLE

A number of typical compounds of the present invention (Compounds 1 to 95) were tested for in vitro human leukocyte elastase inhibiting activity essentially by the method described in JP-A-2-101017, with slight modification.

Thus, methoxysuccinyl-Ala-Ala-Pro-Val-7-amido-4-methylcoumarin was used as a substrate for human leukocyte elastase and the 50% inhibitory concentration ($IC_{50}$) was measured. The enzymatic reaction was carried out in 0.2 M Tris-hydrochloride buffer (containing 0.02 M $CaCl_2$ and 0.005% Triton X-100) at pH 7.2. Each mixture of test compound solution and the enzyme solution was incubated at 25 °C for 20 minutes.

For comparison, the compound 95 according to the following formula [V] was also examined, which was described to have elastase inhibiting activity in JP-A-165357.

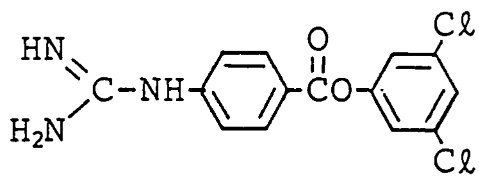

[V]

The results obtained are shown in Table 2.

Table 2

| No | IC50 | No | IC50 | No | IC50 | No | IC50 | No | IC50 |
|---|---|---|---|---|---|---|---|---|---|
| 1 | $3.7 \times 10^{-8}$ | 21 | $1.4 \times 10^{-7}$ | 41 | $4.7 \times 10^{-8}$ | 61 | $9.3 \times 10^{-9}$ | 81 | $6.5 \times 10^{-9}$ |
| 2 | $7.0 \times 10^{-9}$ | 22 | $2.3 \times 10^{-8}$ | 42 | $2.1 \times 10^{-9}$ | 62 | $1.3 \times 10^{-9}$ | 82 | $1.8 \times 10^{-8}$ |
| 3 | $1.9 \times 10^{-9}$ | 23 | $1.4 \times 10^{-7}$ | 43 | $7.6 \times 10^{-9}$ | 63 | $9.7 \times 10^{-8}$ | 83 | $2.5 \times 10^{-8}$ |
| 4 | $2.3 \times 10^{-8}$ | 24 | $9.8 \times 10^{-8}$ | 44 | $1.8 \times 10^{-8}$ | 64 | $2.2 \times 10^{-7}$ | 84 | $2.1 \times 10^{-8}$ |
| 5 | $3.7 \times 10^{-8}$ | 25 | $2.6 \times 10^{-7}$ | 45 | $1.8 \times 10^{-8}$ | 65 | $4.5 \times 10^{-9}$ | 85 | $2.4 \times 10^{-8}$ |
| 6 | $1.1 \times 10^{-7}$ | 26 | $4.7 \times 10^{-8}$ | 46 | $6.7 \times 10^{-9}$ | 66 | $9.4 \times 10^{-8}$ | 86 | $2.4 \times 10^{-8}$ |
| 7 | $4.1 \times 10^{-9}$ | 27 | $2.1 \times 10^{-8}$ | 47 | $3.8 \times 10^{-9}$ | 67 | $8.8 \times 10^{-8}$ | 87 | $1.9 \times 10^{-8}$ |
| 8 | $6.4 \times 10^{-9}$ | 28 | $1.6 \times 10^{-8}$ | 48 | $1.6 \times 10^{-8}$ | 68 | $1.1 \times 10^{-7}$ | 88 | $4.8 \times 10^{-8}$ |
| 9 | $5.3 \times 10^{-9}$ | 29 | $2.0 \times 10^{-8}$ | 49 | $1.8 \times 10^{-7}$ | 69 | $2.5 \times 10^{-8}$ | 89 | $4.0 \times 10^{-7}$ |
| 10 | $2.8 \times 10^{-8}$ | 30 | $1.9 \times 10^{-7}$ | 50 | $4.7 \times 10^{-9}$ | 70 | $2.6 \times 10^{-8}$ | 90 | $1.1 \times 10^{-8}$ |
| 11 | $6.7 \times 10^{-8}$ | 31 | $1.3 \times 10^{-8}$ | 51 | $2.6 \times 10^{-9}$ | 71 | $3.1 \times 10^{-7}$ | 91 | $3.0 \times 10^{-7}$ |
| 12 | $1.0 \times 10^{-8}$ | 32 | $5.9 \times 10^{-9}$ | 52 | $1.5 \times 10^{-8}$ | 72 | $2.1 \times 10^{-8}$ | 92 | $9.1 \times 10^{-8}$ |
| 13 | $9.0 \times 10^{-9}$ | 33 | $2.6 \times 10^{-8}$ | 53 | $3.4 \times 10^{-9}$ | 73 | $1.1 \times 10^{-7}$ | 93 | $3.6 \times 10^{-8}$ |
| 14 | $3.5 \times 10^{-9}$ | 34 | $2.6 \times 10^{-7}$ | 54 | $2.1 \times 10^{-9}$ | 74 | $3.3 \times 10^{-9}$ | 94 | $2.4 \times 10^{-7}$ |
| 15 | $7.7 \times 10^{-9}$ | 35 | $3.8 \times 10^{-8}$ | 55 | $3.6 \times 10^{-9}$ | 75 | $2.5 \times 10^{-9}$ | | |
| 16 | $9.2 \times 10^{-9}$ | 36 | $3.1 \times 10^{-8}$ | 56 | $8.4 \times 10^{-9}$ | 76 | $1.9 \times 10^{-9}$ | | |
| 17 | $1.4 \times 10^{-9}$ | 37 | $7.9 \times 10^{-8}$ | 57 | $2.9 \times 10^{-9}$ | 77 | $3.8 \times 10^{-9}$ | | |
| 18 | $1.0 \times 10^{-8}$ | 38 | $1.8 \times 10^{-8}$ | 58 | $3.5 \times 10^{-9}$ | 78 | $9.6 \times 10^{-9}$ | 95 | $5.7 \times 10^{-7}$ |
| 19 | $9.6 \times 10^{-8}$ | 39 | $9.6 \times 10^{-8}$ | 59 | $2.7 \times 10^{-9}$ | 79 | $1.1 \times 10^{-7}$ | | |
| 20 | $1.2 \times 10^{-8}$ | 40 | $3.4 \times 10^{-9}$ | 60 | $4.2 \times 10^{-9}$ | 80 | $2.4 \times 10^{-8}$ | | |

## Claims

1. A benzoic acid phenylester compound having a substituted amino group at position 4 according to the following formula [I]:

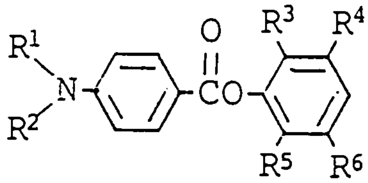

[I]

wherein $R^1$ is a $COR^7$, $CO(CR^8R^9)_nCOOH$, mesyl, benzenesulfonyl, $CONR^{10}R^{11}$, $COOCOR^{12}$ or $COOR^{13}$ group and $R^2$ is a hydrogen atom, or $R^1$ and $R^2$ combinedly represent a $-CO(CR^8R^9)_nCO-$ group for imide ring formation with the adjacent nitrogen atom; and $R^3$, $R^4$, $R^5$ and $R^6$ may be the same or different and each is a hydrogen or halogen atom or a nitrile, nitro, formyl, carboxyl, hydroxyl, $C_{1-4}$ alkoxy, benzyloxy, methyl, aminocarbonyl, trifluoromethyl, trifluoromethoxy, $C_{1-2}$ dialkylaminocarbonyloxy, $C_{1-2}$ alkoxycarbonyl, substituted or unsubstituted benzyloxycarbonyl, straight or branched $C_{1-6}$ alkylcarbonyloxy, alkyl-substituted or unsubstituted $C_{3-6}$ cycloalkylcarbonyloxy, cinnamoyloxy, straight or branched $C_{1-4}$ alkylsulfonyloxy, substituted or unsubstituted benzenesulfonyloxy, benzylsulfonyloxy, $\beta$-stylenesulfonyloxy, 2-thiophenesulfonyloxy, or straight or branched $C_{2-4}$ alkylcarbonyl group, $R^7$ is a straight or branched $C_{2-7}$ alkyl, straight or branched $C_{2-5}$ alkenyl, benzyl, $\alpha$-methylbenzyl, $C_{1-5}$ haloalkyl, alkyl- or carboxyl-substituted or unsubstituted $C_{3-7}$ cycloalkyl, thiol-substituted $C_{1-3}$ alkyl, methylthio-substituted $C_{1-3}$ alkyl, cyano-substituted $C_{1-5}$ alkyl, $\beta$-styryl or (2-thienyl)methyl group, $R^8$ and $R^9$ may be the same or different and each is a hydrogen atom or a straight or branched $C_{1-4}$ alkyl or phenyl group, $R^{10}$ is a straight or branched $C_{1-5}$ alkyl group, $R^{11}$ is a hydrogen atom or a straight or branched $C_{1-4}$ alkyl group, $R^{12}$ is a straight or branched $C_{1-4}$ alkyl group, $R^{13}$ is a hydroxy-substituted or unsubstituted $C_{1-4}$ alkyl, or benzyl group, and n is an integer of 1 to 3.

2. A human leukocyte elastase inhibiting composition which comprises a benzoic acid phenylester compound having a substituted amino group at position 4 and having formula [I]:

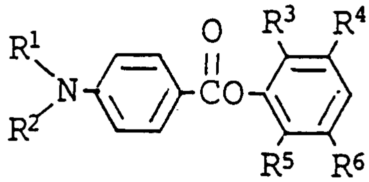

[I]

wherein $R^1$ is a $COR^7$, $CO(CR^8R^9)_nCOOH$, mesyl, benzenesulfonyl, $CONR^{10}R^{11}$, $COOCOR^{12}$ or $COOR^{13}$ group and $R^2$ is a hydrogen atom, or $R^1$ and $R^2$ combinedly represent a $-CO(CR^8R^9)_nCO-$ group for imide ring formation with the adjacent nitrogen atom; and $R^3$, $R^4$, $R^5$ and $R^6$ may be the same or different and each is a hydrogen or halogen atom or a nitrile, nitro, formyl, carboxyl, hydroxyl, $C_{1-4}$ alkoxy, benzyloxy, methyl, aminocarbonyl, trifluoromethyl, trifluoromethoxy, $C_{1-2}$ dialkylaminocarbonyloxy, $C_{1-2}$ alkoxycarbonyl, substituted or unsubstituted benzyloxycarbonyl, straight or branched $C_{1-6}$alkylcarbonyloxy, alkyl-substituted or unsubstituted $C_{3-6}$ cycloalkylcarbonyloxy, cinnamoyloxy, straight or branched $C_{1-4}$ alkylsulfonyloxy, substituted or unsubstituted benzenesulfonyloxy, benzylsulfonyloxy, $\beta$-stylenesulfonyloxy, 2-thiophenesulfonyloxy, or straight or branched $C_{2-4}$ alkylcarbonyl group, $R^7$ is a straight or branched $C_{2-7}$ alkyl, straight or branched $C_{2-5}$ alkenyl, benzyl, $\alpha$-methylbenzyl, $C_{1-5}$ haloalkyl, alkyl- or carboxyl-substituted or unsubstituted $C_{3-7}$ cycloalkyl, thiol-substituted $C_{1-3}$ alkyl, methylthio-substituted $C_{1-3}$ alkyl, cyano-substituted $C_{1-5}$ alkyl, $\beta$-styryl or (2-thienyl)methyl group, $R^8$ and $R^9$ may be the same or different and each is a hydrogen atom or a straight or branched $C_{1-4}$ alkyl or phenyl group, $R^{10}$ is a straight or branched $C_{1-5}$ alkyl group, $R^{11}$ is a hydrogen atom or a straight or branched $C_{1-4}$ alkyl group, $R^{12}$ is a straight or branched $C_{1-4}$ alkyl group, $R^{13}$ is a hydroxy-substituted or unsubstituted $C_{1-4}$ alkyl, or benzyl group, and n is an integer of

1 to 3.

**Patentansprüche**

1. Eine Benzoesäurephenylester-Verbindung mit einer substituierten Aminogruppe in 4-Stellung der folgenden Formel [I]:

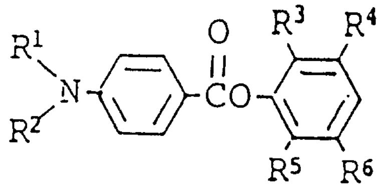

[I]

worin $R^1$ eine $COR^7$-, $CO(CR^8R^9)_nCOOH$, Mesyl-, Benzolsulfonyl-, $CONR^{10}R^{11}$-, $COOCOR^{12}$ - oder $COOR^{13}$-Gruppe und $R^2$ ein Wasserstoffatom ist oder $R^1$ und $R^2$ zusammen eine $-CO(CR^8R^9)_nCO$-Gruppe für eine Imidringbildung mit dem benachbarten Stickstoffatom darstellen, und $R^3$, $R^4$, $R^5$ und $R^6$ gleich oder verschieden sein können und jeweils ein Wasserstoff- oder Halogenatom oder eine Nitril-, Nitro-, Formyl-, Carboxyl-, Hydroxyl-, $C_{1-4}$-Alkoxy-, Benzyloxy-, Methyl-, Aminocarbonyl-, Trifluormethyl-, Trifluormethoxy-, $C_{1-2}$-Dialkylaminocarbonyloxy-,$C_{1-2}$-Alkoxycarbonyl-, substituierte oder unsubstituierte Benzyloxycarbonyl-, geradkettige oder verzweigte $C_{1-6}$-Alkylcarbonyloxy-, alkylsubstituierte oder unsubstituierte $C_{3-6}$-Cycloalkylcarbonyloxy-,Zinnamoyloxy-, geradkettige oder verzweigte $C_{1-4}$-Alkylsulfonyloxy-, substituierte oder unsubstituierte Benzolsulfonyloxy-, Benzylsulfonyloxy-, $\beta$-Stylensulfonyloxy-, 2-Thiophensulfonyloxy- oder geradkettige oder verzweigte $C_{2-4}$-Alkylcarbonyl-Gruppe bedeuten, $R^7$ eine geradkettige oder verzweigte $C_{2-7}$-Alkyl-, geradkettige oder verzweigte $C_{2-5}$-Alkenyl-, Benzyl-, $\alpha$-Methylbenzyl-, $C_{1-5}$-Haloalkyl-, alkyl- oder carboxylsubstituierte oder unsubstituierte $C_{3-7}$-Cycloalkyl-, thiol-substituierte $C_{1-3}$-Alkyl-, methylthiosubstituierte $C_{1-3}$-Alkyl-, cyanosubstituierte $C_{1-5}$-Alkyl-, $\beta$-Styryl- oder (2-Thienyl)-methyl-Gruppe ist, $R^8$ und $R^9$ gleich oder verschieden sein können und jeweils ein Wasserstoffatom oder eine geradkettige oder verzweigte $C_{1-4}$-Alkyl- oder eine Phenyl-Gruppe bedeuten, $R^{10}$ eine geradkettige oder verzweigte $C_{1-5}$-Alkyl-Gruppe bedeutet, $R^{11}$ ein Wasserstoffatom oder eine geradkettige oder verzweigte $C_{1-4}$-Alkyl-Gruppe ist, $R^{12}$ eine geradkettige oder verzweigte $C_{1-4}$-Alkylgruppe ist, $R^{13}$ eine hydroxysubstituierte oder unsubstituierte $C_{1-4}$-Alkyl- oder eine Benzyl-Gruppe ist und n eine ganze Zahl von 1 bis 3 ist.

2. Eine human-leukozytenelastaseinhibierende Zusammensetzung mit einer Benzoesäurephenylester-Verbindung mit einer substituierten Aminogruppe in 4-Stellung und mit der Formel [I]:

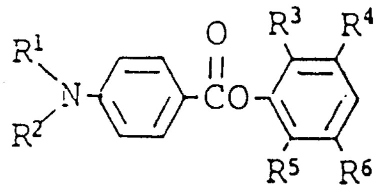

[I]

worin $R^1$ eine $COR^7$-, $CO(CR^8R^9)_nCOOH$, Mesyl-, Benzolsulfonyl-, $CONR^{10}R^{11}$-, $COOCOR^{12}$- oder $COOR^{13}$-Gruppe und $R^2$ ein Wasserstoffatom ist oder $R^1$ und $R^2$ zusammen eine $-CO(CR^8R^9)_nCO$-Gruppe für eine Imidringbildung mit dem benachbarten Stickstoffatom darstellen, und $R^3$, $R^4$, $R^5$ und $R^6$ gleich oder verschieden sein können und jeweils ein Wasserstoff- oder Halogenatom oder eine Nitril-, Nitro-, Formyl-, Carboxyl-, Hydroxyl-, $C_{1-4}$-Alkoxy-, Benzyloxy-, Methyl-, Aminocarbonyl-, Trifluormethyl-, Trifluormethoxy-, $C_{1-2}$-Dialkylaminocarbonyloxy-,$C_{1-2}$-Alkoxycarbonyl-, substituierte oder unsubstituierte Benzyloxycarbonyl-, geradkettige oder verzweigte $C_{1-6}$-Alkylcarbonyloxy-, alkylsubstituierte oder unsubstituierte $C_{3-6}$-Cycloalkylcarbonyloxy-,Zinnamoyloxy-, geradkettige oder verzweigte $C_{1-4}$-Alkylsulfonyloxy-, substituierte oder unsubstituierte Benzolsulfonyloxy-, Benzylsulfonyloxy-, $\beta$-Stylensulfonyloxy-, 2-Thiophensulfonyloxy- oder geradkettige oder verzweigte $C_{2-4}$-Alkylcarbonyl-Gruppe bedeuten, $R^7$ eine geradkettige oder verzweigte $C_{2-7}$-Alkyl-, geradkettige oder verzweigte $C_{2-5}$-Alkenyl-, Benzyl-, $\alpha$-Methylbenzyl-, $C_{2-5}$-Haloalkyl-, alkyl- oder carboxylsubstituierte oder unsubstituier-

te $C_{3-7}$-Cycloalkyl-, thiol-substituierte $C_{1-3}$-Alkyl-, methylthiosubstituierte $C_{1-3}$-Alkyl-, cyanosubstituierte $C_{1-5}$-Alkyl-, $\beta$-Styryl- oder (2-Thienyl)-methyl-Gruppe ist, $R^8$ und $R^9$ gleich oder verschieden sein können und jeweils ein Wasserstoffatom oder eine geradkettige oder verzweigte $C_{1-4}$-Alkyl- oder eine Phenyl-Gruppe bedeuten, $R^{10}$ eine geradkettige oder verzweigte $C_{1-5}$-Alkyl-Gruppe bedeutet, $R^{11}$ ein Wasserstoffatom oder eine geradkettige oder verzweigte $C_{1-4}$-Alkyl-Gruppe ist, $R^{12}$ eine geradkettige oder verzweigte $C_{1-4}$-Alkylgruppe ist, $R^{13}$ eine hydroxysubstituierte oder unsubstituierte $C_{1-4}$-Alkyl- oder eine Benzyl-Gruppe ist und n eine ganze Zahl von 1 bis 3 ist.

## Revendications

1. Dérivé d'esters phényliques de l'acide benzoïque ayant un groupe amino substitué en position 4 conformément à la formule suivante [I] :

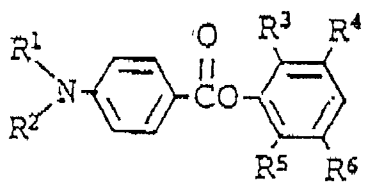

[I]

où $R^1$ est un groupe $COR^7$, $CO(CR^8R^9)_nCOOH$, mésyle, benzène sulfonyle, $CONR^{10}R^{11}$, $COOCOR^{12}$ ou $COOR^{13}$ et $R^2$ est un atome d'hydrogène ou $R^1$ et $R^2$ combinés représentent un groupe - $CO(CR^8R^9)_n$CO- pour la cyclisation d'une imide avec l'atome d'azote adjacent ; et $R^3$, $R^4$, $R^5$ et $R^6$ peuvent être identiques ou différents et chacun est un atome d'halogène ou d'hydrogène ou un groupe nitrile, nitro, formyle, carboxyle, hydroxyle, alcoxy $C_{1-4}$, benzyloxy, méthyle, aminocarbonyle, trifluorométhyle, trifluorométhoxy, dialkylaminocarbonyloxy $C_{1-2}$, alcoxycarbonyle $C_{1-2}$, benzyloxycarbonyle substitué ou non, alkylcarbonyloxy $C_{1-6}$ linéaire ou ramifié, cycloalkylcarbonyloxy $C_{3-6}$ substitué par un groupe alkyle ou non, cinnamoyloxy, alkylsulfonyloxy $C_{1-4}$ linéaire ou ramifié, benzènsulfonyloxy substitué ou non, benzylsulfonyloxy, $\beta$-stylènsulfonyloxy, 2-thiophènesulfonyloxy, ou alkylcarbonyle $C_{2-4}$ linéaire ou ramifié, $R^7$ est un groupe alkyle $C_{2-7}$ linéaire ou ramifié, alcényle $C_{2-5}$ linéaire ou ramifié, benzyle, $\alpha$-méthylbenzyle, haloalkyle $C_{1-5}$, cycloalkyle $C_{3-7}$ substitué par un groupe alkyle ou carboxyle ou non, alkyle $C_{1-3}$ substitué par un groupe thiol, alkyle $C_{1-3}$ substitué par un groupe méthylthio, alkyle $C_{1-5}$ substitué par un groupe cyano, (2-thiényl)méthyle ou $\beta$-styryle, $R^8$ ou $R^9$ peuvent être égaux ou différents et chacun est un atome d'hydrogène ou un groupe phényle ou alkyle $C_{1-4}$ linéaire ou ramifié, $R^{10}$ est un groupe alkyle $C_{1-5}$ linéaire ou ramifié, $R^{11}$ est un atome d'hydrogène ou un groupe alkyle $C_{1-4}$ linéaire ou ramifié, $R^{12}$ est un groupe alkyle $C_{1-4}$ linéaire ou ramifié, $R^{13}$ est un groupe alkyle $C_{1-4}$ substitué par un groupe hydroxy ou non, ou un groupe benzyle et n est un nombre entier valent de 1 à 3.

2. Composition inhibitrice de l'élastase leucocytaire humaine qui comprend un dérivé d'esters phényliques de l'acide benzoïque ayant un groupe amino substitué en position 4 et répondant à la formule [I] :

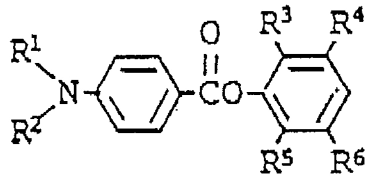

[I]

où $R^1$ est groupe $COR^7$, $CO(CR^8R^9)_nCOOH$, mésyle, benzène sulfonyle, $CONR^{10}R^{11}$, $COOCOR^{12}$ ou $COOR^{13}$ et $R^2$ est un atome d'hydrogène ou $R^1$ et $R^2$ combinés représentent un groupe - $CO(CR^8R^9)_n$CO- pour la cyclisation d'une imide avec l'atome d'azote adjacent ; et $R^3$, $R^4$, $R^5$ et $R^6$ peuvent être identiques ou différents et chacun est un atome d'halogène ou d'hydrogène ou un groupe nitrile, nitro, formyle, carboxyle, hydroxyle, alcoxy $C_{1-4}$, benzyloxy, méthyle, aminocarbonyle, trifluorométhyle, trifluorométhoxy, dialkylaminocarbonyloxy $C_{1-2}$, alcoxycarbonyle $C_{1-2}$, benzyloxycarbonyle substitué ou non, alkylcarbonyloxy $C_{1-6}$ linéaire ou ramifié, cycloalkylcarbonyloxy $C_{3-6}$ substitué par un groupe

alkyle ou non, cinnamoyloxy, alkylsulfonyloxy $C_{1-4}$ linéaire ou ramifié, benzènsulfonyloxy substitué ou non, benzylsulfonyloxy, $\beta$-stylènsulfonyloxy, 2-thiophènesulfonyloxy, alkylcarbonyle $C_{2-4}$ linéaire ou ramifié, $R^7$ est un groupe alkyle $C_{2-7}$ linéaire ou ramifié, alcényle $C_{2-5}$ linéaire ou ramifié, benzyle, $\alpha$-méthylbenzyle, haloalkyle $C_{1-5}$, cycloalkyle $C_{3-7}$ substitué par un groupe alkyle ou carboxyle ou non, alkyle $C_{1-3}$ substitué par un groupe thiol, alkyle $C_{1-3}$ substitué par un groupe méthylthio, alkyle $C_{1-5}$ substitué par un groupe cyano (2-thiényl)méthyle ou $\beta$-styryle, $R^8$ ou $R^9$ peuvent être égaux ou différents et chacun est un atome d'hydrogène ou un groupe phényle ou alkyle $C_{1-4}$ linéaire ou ramifié, $R^{10}$ est un groupe alkyle $C_{1-5}$ linéaire ou ramifié, $R^{11}$ est un atome d'hydrogène ou un groupe alkyle $C_{1-4}$ linéaire ou ramifié, $R^{12}$ est un groupe alkyle $C_{1-4}$ linéaire ou ramifié, $R^{13}$ est un groupe alkyle $C_{1-4}$ substitué par un groupe hydroxy ou non, ou un groupe benzyle et n est un nombre entier valant de 1 à 3.